Europäisches Patentamt

European Patent Office

Office européen des brevets

(19)

(11) Numéro de publication: **0 032 349**
**B1**

(12) **FASCICULE DE BREVET EUROPEEN**

(45) Date de publication du fascicule du brevet: **27.07.83**

(51) Int. Cl.³: **A 61 M 16/00**

(21) Numéro de dépôt: **80401883.6**

(22) Date de dépôt: **29.12.80**

(54) Dispositif de commande pour respirateur artificiel.

(30) Priorité: **04.01.80 FR 8000096**

(43) Date de publication de la demande:
**22.07.81 Bulletin 81/29**

(45) Mention de la délivrance du brevet:
**27.07.83 Bulletin 83/30**

(84) Etats contractants désignés:
**AT BE CH DE FR GB IT LI LU NL SE**

(56) Documents cités:
**GB - A - 2 000 443**

(73) Titulaire: **SYNTHELABO**
**58 rue de la Glacière**
**F-75013 Paris (FR)**

(72) Inventeur: **Caillot, Luc**
**39, rue de l'Amiral Mouchez**
**F-75013 Paris (FR)**

(74) Mandataire: **Pinguet, André**
**CAPRI 28 bis, avenue Mozart**
**F-75016 Paris (FR)**

Courier Press, Leamington Spa, England.

## Dispositif de commande pour respirateur artificiel

La présente invention a trait à une respirateur artificiel et a pour objet un dispositif de commande électronique pour un tel respirateur.

On sait qu'un tel appareil est utilisé pour la respiration forcée d'un patient, notamment pendant qu'il est sous anesthésie au cours d'une opération ou en réanimation ou en unité de soins intensifs par exemple. L'appareil a pour fonction de fournir au patient de l'air souvent enrichi en oxygène, donc un mélange d'air et d'oxygène, à une cadence correspondant à celle de la respiration et à une légère surpression (quelques dizaines de millibar, de façon à suppléer ou renforcer la fonction déficiente). Il est important de pouvoir choisir avec précision le débit du mélange de gaz à envoyer au patient, la teneur en oxygène de ce mélange, la période T de la respiration qui lui est imposée, et dans cette période, la partie inspiration et la partie expiration (donc le rapport cyclique I/E).

Jusqu'à présent, on utilise typiquement un dispositif comportant un mélangeur mécanique, suivi d'un pointeau de réglage du débit, le mélange sortant passant par une électrovanne commandée par une base de temps. L'inconvénient principal d'un tel système est que le débit est modifié si l'on agit sur la période, ou sur la teneur en $O_2$ du mélange, et qu'il est par conséquent long et difficile d'obtenir des réglages précis, qui sont en particulier très difficiles à modifier au cours d'une opération.

La présente invention a pour objet un dispositif de commande dont les paramètres peuvent être réglés de façon totalement indépendante.

Selon la présente invention un dispositif de commande pour respirateur artificiel comprend: une électrovanne d'air et une électrovanne d'oxygène, du type à débit variable en fonction de la course, débitant dans un collecteur; une base de temps avec un organe de fixation de la fréquence ou de la période respiratoire et un organe de fixation du rapport du temps d'inspiration au temps d'expiration pendant la période de la respiration; un mélangeur, comportant des organes de fixation du débit respiratoire global et de la proportion d'oxygène du gaz fourni par le respirateur; et deux générateurs de signaux affectés chacun à une des électrovannes pour commander leurs ouvertures; la base de temps étant connectée aux générateurs pour qu'ils commandent l'ouverture des vannes pendant le temps d'inspiration, les générateurs étant connectés par ailleurs au mélangeur pour recevoir les signaux correspondant aux amplitudes des ouvertures des vannes pendant le temps d'inspiration, le mélangeur étant connecté à la base du temps pour recevoir un signal correspondant au rapport du temps d'inspiration au temps d'expiration de façon à élaborer avec les indications de débit et de proportion, des signaux pour les ouvertures des vannes, tels que pendant le temps inspiration, chaque vanne laisse passer une quantité de gaz correspondant au débit moyen pendant la période de la respiration.

La description qui va suivre en regard des dessins annexés, donnée à titre d'exemple non limitatif, fera bien comprendre comment l'invention peut être réalisée.

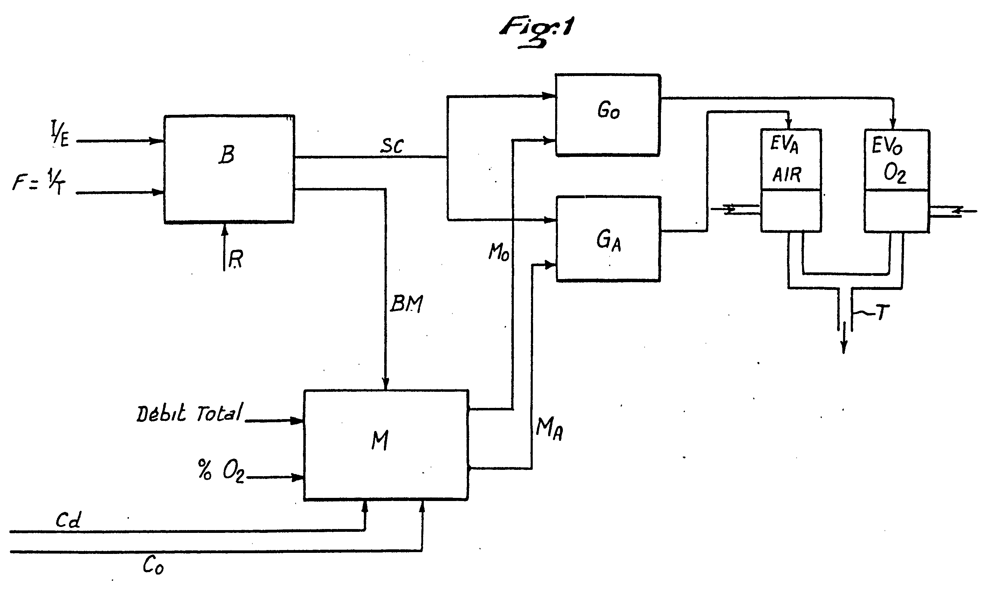

La figure 1 est un schéma bloc du dispositif selon l'invention,

La figure 2 est un schéma de montage d'un élément du dispositif de la figure 1 (Mélangeur M),

La figure 3 est un schéma de montage d'un autre élément du dispositif de la figure 1 (Générateur G),

La figure 4 est une vue en coupe schématique d'un autre élément constitué par une vanne et,

La figure 5 est un exemple d'un schéma de montage complet du circuit électronique avec indication des éléments et de leurs valeurs.

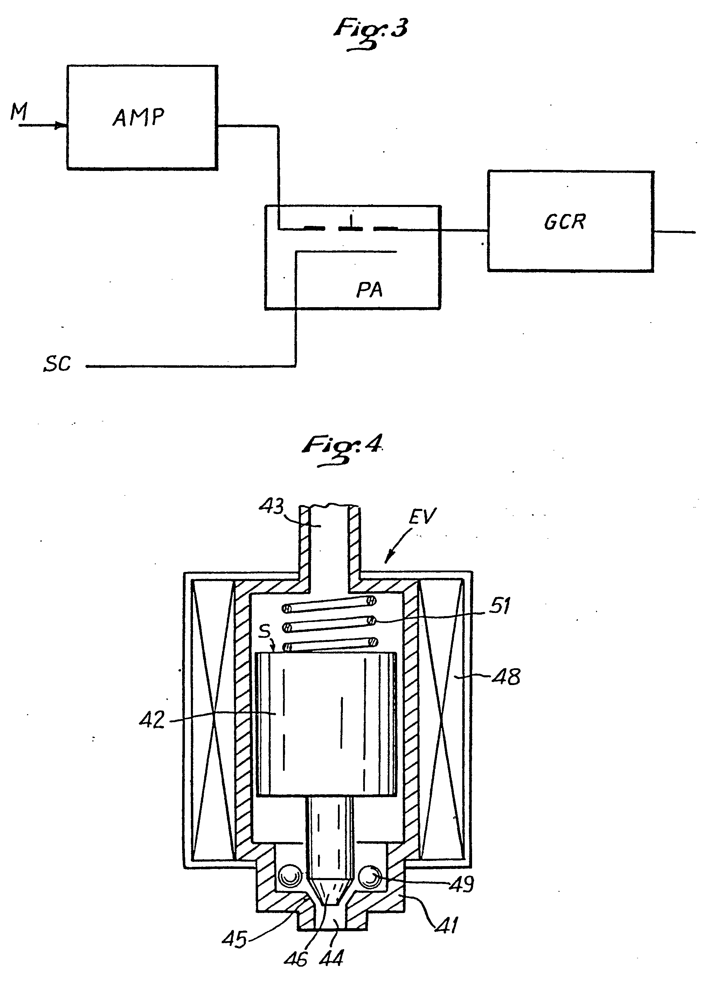

Le dispositif de commande du respirateur artificiel comporte une électrovanne d'air $EV_A$ et une électrovanne d'oxygène EVO. Ces électrovannes (voir figure 4 et description en regard) sont du type à ouverture variable en fonction des ampère-tours de commande; elles sont alimentées en air et en oxygène sous des pressions régulées très précises. Le débit d'air et d'oxygène est donc une fonction précise du temps d'ouverture et de la course du noyau de chaque électrovanne. Les électrovannes débitent dans un tube T, directement branché sur le circuit d'alimentation en gaz vers le patient, et faisant partie du respirateur, qui, en lui-même, est d'un type classique quelconque.

La commande d'ouverture de chaque électrovanne est assurée par deux générateurs de signaux $G_A$ et $G_O$ identiques, chacun commandant une électrovanne. Les générateurs $G_A$ et $G_O$ sont commandés eux-mêmes par une base de temps B, comportant des moyens pour permettre à l'opération de fixer d'une part la fréquence

$$F = \frac{1}{T}$$

de la respiration, d'autre part, le rapport cyclique I/E (E + I = T) du temps d'inspiration au temps d'expiration et, par un mélangeur M comportant des moyens de réglage du débit total de mélange air-oxygène à envoyer au patient, et de la proportion d'oxygène dans ce mélange. La base de temps peut en outre être automatiquement réajustée par une réaction R, provenant par exemple de mesures prises sur la patient.

La base de temps est connectée par une ligne SC aux deux générateurs, pour leur injecter le même signal chronologique. A partir de ces signaux, les générateurs commandent l'ouverture des électrovannes pendant le temps I, dans chaque période de temps T.

Les amplitudes d'ouverture des électrovannes sont élaborées par les générateurs à partir de signaux reçus du mélangeur par les lignes $M_O$ et $M_A$. Ces signaux de commande tiennent compte du débit du mélange et de la proportion d'oxygène, deux paramètres qui sont choisis par l'opérateur et d'un signal lié à I/E, envoyé par la base de temps B au mélangeur M par la ligne BM. Ont peut ainsi obtenir un débit, avec un pourcentage en oxygène, qui soit fixe, quelles que soient les valeurs choisies par ailleurs par l'opérateur pour F et I/S.

Le mélangeur peut en outre recevoir des signaux extérieurs de correction du débit et du pourcentage en oxygène, par des lignes Cd et Co, par exemple dans le cas d'une surveillance automatique de certains paramètres du patient.

Considérons les paramètres à déterminer. Si l'on appelle $D_T$ le débit total, air + oxygène, $D_O$ et $D_A$ les débits d'oxygène et d'air respectivement on a:

$$D_T = D_O + D_A \qquad (1)$$

et si on appelle $F_O$ le pourcentage d'oxygène dans le mélange on a:

$$F_O = \frac{D_O + 0,21\, D_A}{D_O + D_A} \qquad (2)$$

Le mélangeur est constitué de telle manière que l'on puissé régler indépendamment l'une et l'autre $D_T$ et $F_O$. La génération des signaux électriques est réalisée conformément à la présente invention à partir des égalités (1) et (2) ci-dessus. La génération des signaux de commande peut être établie aussi bien par un procédé analogique que totalement numérique. Ce dernier procédé peut être préféré dans le cas d'un pilotage automatique des paramètres à partir d'une source extérieure de régulation.

A partir de l'agalité 2, on peut écrire:

$$D_A = D_O \; \frac{(1 - F_O)}{F_O - 0,21} = D_O \cdot K$$

d'où l'on tire pour exprimer $D_O$ et $D_A$ en fonction de $D_T$:

$$D_O = \frac{D_T}{1 + K} \qquad D_A = D_T \; \frac{K}{1 + K}$$

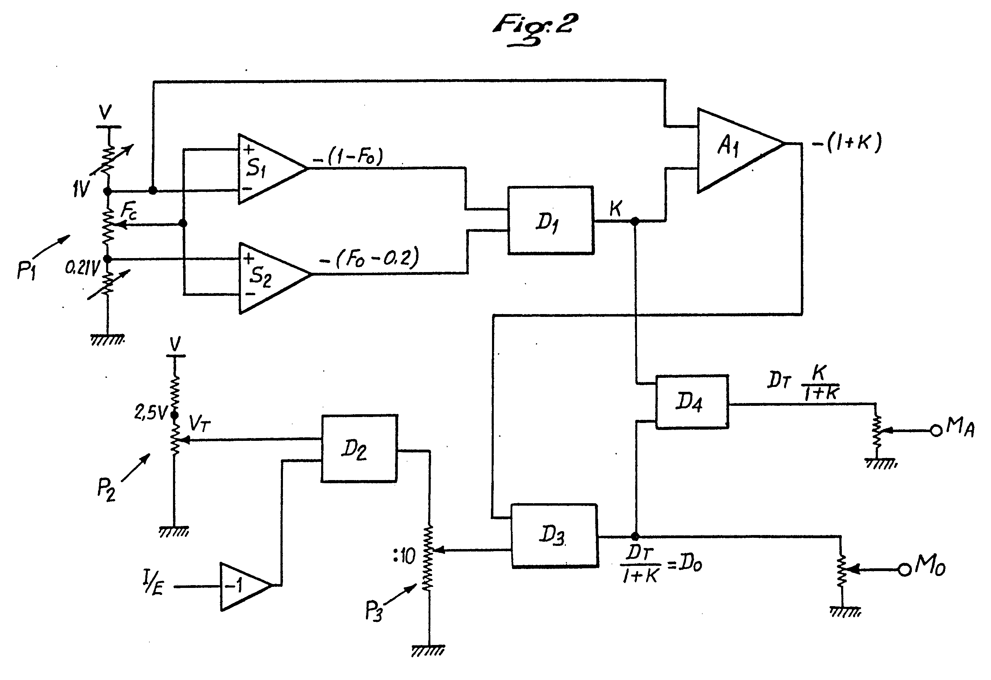

Selon une caractéristique de l'invention, la génération des signaux est tirée de ces formules. Une réalisation pratique préférée est preprésentée sur la figure 2. Un premier potentiomètre $P_1$ est utilisé pour la fixation par l'opérateur du pourcentage en oxygène $F_O$ du mélange. On utilise une source de tension stabilisée V avec deux résistances de réglage, et, à l'aide du potentiomètre $P_1$, on choisit une tension $F_O$ comprise entre 0,21 volt et 1 volt, représentant la proportion d'oxygène comprise entre 0,21 air pur et 1 oxygène pur. Au moyen de deux soustracteurs $S_1$ et $S_2$, on obtient à partir des trois tensions 0,21, $F_O$ et 1 volt, les tensions représentatives de $-(1 - F_O)$ et $-(F_O = 0,21)$ que l'on injecte dans le diviseur $D_1$ pour obtenir:

$$\frac{1 - F_O}{F_O - 0,21}$$

c'est-à-dire K. (Pour des raisons de commodités pratiques, on peut utiliser pour $S_2$ un soustracteur de rapport $-10$ et pour D un diviseur à amplification de 10).

Pour obtenir 1 + K, la valeur K, soit directement, soit après injection dans un multiplicateur recevant une tension de 1 volt, est injectée sur une des entrées d'un additionneur inverseur $A_1$, la tension de 1 volt étant injectée sur l'autre entrée. La sortie de l'additionneur $A_1$ donne une valeur égale alors à $-(1 + K)$.

Un deuxième potentiomètre $P_2$ est utilisé pour fixer le débit total. Au moyen d'une résistance et d'une source de tension stabilisée V, on délimite la plage de réglage $P_2$ entre 2,5 volts et 0 et l'opérateur peut ainsi choisir une tension $V_T$ représentative du débit total de mélange air + oxygène

Un signal directement proportionnel 1 à I/E issu de la base de temps B, arrivant par la ligne BM passe dans un inverseur $(-1)$, puis le signal $V_T$ et le signal $-I/E$ sont injectés dans un diviseur $D_2$ de façon à avoir à la sortie un signal proportionnel au débit que l'on devra avoir pendant le temps de l'ouverture de l'électrovanne, c'est-à-dire pendant la période d'inspiration. Grâce à cette opération, le débit total fixé est toujours indépendant du rapport I/E choisi. L'opérateur fixe donc le potentiomètre $P_2$ le débit désiré, sans s'occuper du rapport I/E et, le diviseur $D_2$ détermine le débit instantané en fonction du rapport I/E.

Le signal de sortie du diviseur $D_2$ est envoyé par un potentiomètre $P_3$ d'ajustage d'échelle dans une diviseur $D_3$ qui reçoit par ailleurs le signal $-(1 + K)$. Il en sort le signal

$$\frac{D_T}{1 + K}$$

c'est-à-dire $D_O$ que l'on prend au contact $M_O$ après un réglage à potentiomètre.

Le signal

$$\frac{D_T}{1 + K}$$

est en outre injecté dans un autre diviseur $D_4$ recevant le signal K sur l'autre entrée, duquel sort le signal

$$D_T \frac{K}{1 + K}$$

c'est-à-dire $D_A$ que l'on prend en $M_A$ après un réglage au potentiomètre.

La figure 3 représente un schéma d'un générateur de signaux analogues faisant le produit du signal de découpage dans le temps fourni par la base de temps et du signal fourni par le mélangeur de gaz, de façon à fournir des signaux comportant trois ordres: fréquence, rapport cyclique, amplitude. Il est rappelé que les deux générateurs $G_0$ et $G_A$ sont identiques. Un générateur comporte un relais à commutation rapide ou une porte analogique PA, ou tout système équivalent, commandée par la base de temps (ligne SC). Le signal M ($M_0$ ou $M_A$) envoyé par le mélangeur est amplifié dans l'amplificateur AMP avant d'être injecté dans le relais. La sortie du relais traverse un générateur de courant régulé GCR avant d'attaquer l'électrovanne EV.

La figure 4 représente en coupe schématique un exemple d'électrovanne utilisable dans la dispositif de la présente invention. L'électrovanne EV comporte un boîtier 41 dans lequel coulisse un noyau 42. Le boîtier comporte une entrée de gaz 43 et une sortie 44 dans laquelle est prévu un siège de clapet 45. Un pointeau 46 coopère avec le siège pour fermer ou ouvrir plus ou moins la vanne en fonction du nombre d'ampère-tours dans la bobine 48. La dimension extérieure du noyau et la dimension intérieure de boîtier sont définies avec précision l'une par rapport à l'autre pour laisser un passage périphérique pour le gaz qui est admis en 43 avec une pression fixée avec précision. Des billes 49 assurent le centrage du pointeau 46. On peut prévoir un ressort 51 pour le rappel en position fermée de la vanne. En général la pression du gaz sur la face supérieur $s$ du noyau 42 suffit à maintenir l'électrovanne fermée en l'absence d'excitation de la bobine.

En fonction du débit affiché, et de la proportion d'oxygène et en fonction du rapport I/E, le noyau 42 est déplacé vers l'entrée 43, d'une distance telle qu'il s'établisse un débit de gaz (air ou oxygène) par la sortie 46 pendant la phase inspiration qui laisse passer une quantité de gaz correspondant au débit moyen pendant la période I + E. On réalise bien ainsi les objectifs visés par la présente invention.

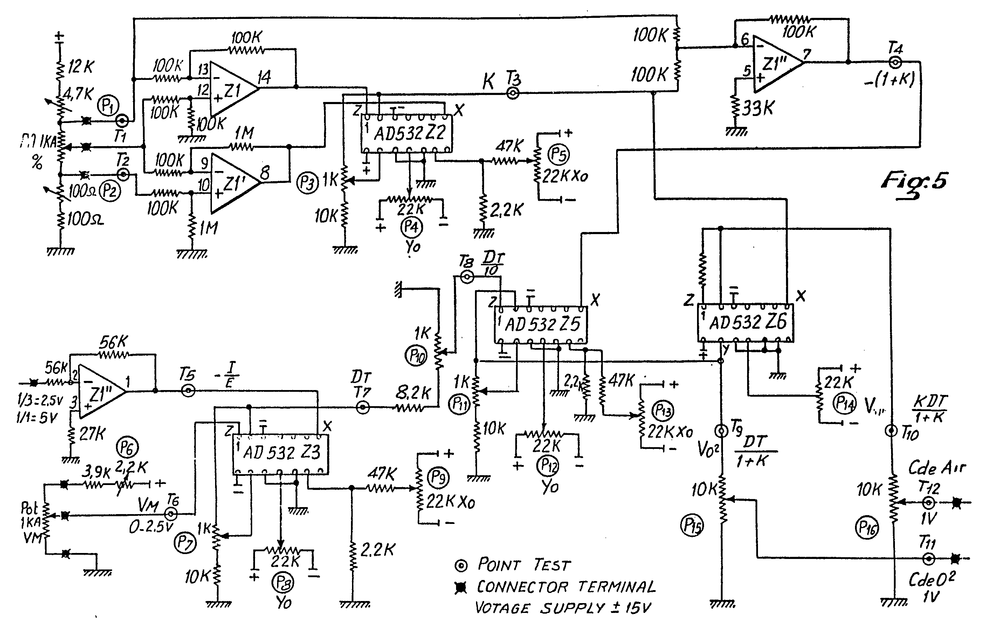

La figure 5 est un exemple d'un schéma de montage complet du circuit électronique avec indication des éléments et de leurs valeurs.

Il va de soi que le mode de réalisation décrit n'est qu'un exemple et qu'il serait possible de le modifier notamment par substitution d'équivalents techniques, sans sortir pour cela du cadre de l'invention.

## Revendications

1. Dispositif de commande pour respirateur artificiel, caractérisé en ce qu'il comprend : une électrovanne d'air (EVA) et une électrovanne d'oxygène ($EV_0$), du type à débit variable en fonction de la course, débitant dans un collecteur (T); une base de temps (B) avec un organe de fixation de la fréquence (F) respiratoire et un organe de fixation du rapport du temps d'inspiration au temps d'expiration (I/E) pendant la période de la respiration (I + E); un mélangeur (M) comportant des organes de fixation du débit respiratoire global et de la proportion d'oxygène du gas fourni par la respirateur; et deux générateurs de signaux ($G_A$, $G_0$) affectés chacun à une des électrovannes ($EV_A$, $EV_0$) pour commander leurs ouvertures; la base de temps (B) étant connectée aux générateurs ($G_A$, $G_0$) pour qu'ils commandent l'ouverture des vannes ($EV_A$, $EV_0$) pendant le temps d'inspiration (I), les générateurs ($G_A$, $G_0$) par ailleurs étant connectés au mélangeur (M) pour recevoir les signaux ($M_A$, $M_0$) correspondant aux amplitudes des ouvertures des vannes ($EV_A$, $EV_0$) pendant le temps d'inspiration (I), le mélangeur (M) étant connecté à la base de temps (B) pour recevoir un signal (BM) correspondant au rapport du temps d'inspiration ou temps d'expiration (I/E) de façon à élaborer avec les indications de débit et de proportion, des signaux pour les ouvertures de vannes ($EV_A$, $EV_0$), tels que pendant le temps d'inspiration (I), chaque vanne ($EV_A$, $EV_0$) laisse passer une quantité de gaz correspondant au débit moyen pendant la période de la respiration.

2. Dispositif selon la revendication 1, caractérisé en ce que chaque générateur ($G_A$ $G_0$) comporte un relais à commutation rapide du type porte analogique, dont l'ouverture est commandée par la base de temps (B).

3. Dispositif selon une des revendications 1 ou 2, caractérisé en que les organes du mélangeur (M) pour fixer le débit et la proportion d'oxygène sont des potentiomètres ($P_1$ $P_2$) montés de façon à délivrer des tensions proportionnelles aux débits d'air et d'oxygène désiré, et que le montage est du type analogique, permettant d'obtenir des grandeurs représentatives du débit total, et à partir de celles-ci du débit d'air et du débit d'oxygène pendant le temps d'inspiration.

4. Dispositif selon une des revendications 1 ou 2, caractérisé en ce que les organes du mélangeur (M) pour fixer le débit et la proportion d'oxygène sont des potentio-mètres ($P_1$ $P_2$) montés de façon à délivrer des tensions proportionnelles aux débits d'air et d'oxygène désiré, et que le montage est du type logique, permettant d'obtenir des grandeurs représentatives du débit total, et à partir de celles-ci du débit d'air et du débit d'oxygène pendant le temps d'inspiration.

## Claims

1. An artificial respirator control device, characterised in that it comprises; a manifold (T) fed from an electrically controlled air valve ($EV_A$) and an electrically controlled oxygen valve ($EV_O$), of the type whose flow varies as a function travel; a time base (B) with a unit for fixing a frequency of breathing and a unit for fixing the ratio of inhaling time to exhaling time (I/E) during a breathing period (I+E); a mixer including units for fixing the overall breathing rate and the proportion of oxygen in the gas supplied by the respirator; and two signal generators ($G_A$, $G_O$) associated with respective ones of the electrically controlled valves ($EV_A$, $EV_O$) for controlling the opening thereof; the time base (B) being so connected to the generators ($G_A$, $G_O$) that they cause the valves ($EV_A$, $EV_O$) to open during the inhaling time, the generators ($G_A$, $G_O$) being further connected to the mixer (M) to receive signals ($M_A$, $M_O$) corresponding to the amplitudes of valve opening during inhaling time (I), the mixer (M) being connected to the time base (B) to receive a signal (BM) corresponding to the ratio of inhaling time to exhaling time (I/E) in such a manner as to generate, in conjunction with the indications of flow rate and of proportion, signals for opening the valves ($EV_A$, $EV_O$), such that during the inhaling time (I), each valve ($EV_A$, $EV_O$) passes a quantity of gas corresponding to the average flow rate during the breathing period.

2. A device according to claim 1, characterised in that each generator ($G_A$, $G_O$) includes a high speed switching relay of the analog gate type, whose opening is controlled by the time base (B).

3. A device according to claim 1 or 2, characterised in that the units of the mixer (M) for fixing the flow rate and the proportion of oxygen are potentiometers ($P_1$, $P_2$) connected to deliver voltages proportional to the desired rates of air and oxygen flow, and that the assembly is of the analog type, enabling values representative of the total flow rate to be obtained, and hence values representative of the rates of air and of oxygen flow during the inhaling times.

4. A device according to claim 1 or 2, characterised in that the units of the mixer (M) for fixing the flow rate and the proportion of oxygen are potentiometers ($P_1$, $P_2$) connected to deliver voltages proportional to the desired rates of air and oxygen flow, and that the assembly is of the logic type, enabling values representative of the total flow rate to be obtained, and hence values representative of the rates of air and of oxygen flow during the inhaling times.

## Patentansprüche

1. Steuervorrichtung für Dauerbeatmungsgerät, dadurch gekennzeichnet, dass sie folgende Teile enthält: ein Magnetventil für Luft ($EV_A$) und eine Magnetventil für Sauerstoff ($EV_O$) vom Typ mit in Abhängigkeit vom Weg veränderlicher Durchflussleistung, die in eine Sammelvorrichtung (T) fördern; eine Zeitbasis (B) mit einem Organ zur Bestimmung der Beatmungsfrequenz (F) und einem Organ zur Bestimmung des Verhältnisses (I/E) der Einatmungszeit zur Ausatmungszeit während der Beatmungsperiode (I + E); einen Mischer (M) mit Organen zur Bestimmung der gesamten Beatmungsleistung und des Sauerstoffanteils im Gas aus dem Dauerbeatmungsgerät; sowie zwei Signalgeber ($G_A$, $G_O$), die je einem der Magnetventile ($EV_A$, $EV_O$) zur Steuerung ihrer Öffnungen zugewiesen sind; wobei die Zeitbasis (B) und die Signalgeber ($G_A$, $G_O$) angeschlossen ist, damit sie das Öffnen der Magnetventile ($EV_A$, $EV_O$) während der Einatmungszeit (I) steuern, wobei die Signalgeber ($G_A$, $G_O$) ferner an den Mischer angeschlossen sind, um die Signale ($M_A$, $M_O$) zu erhalten, die den Öffnungsamplituden der Magnetventile ($EV_A$, $EV_O$) während der Einatmungszeit (I) entsprechen, wobei der Mischer (M) an die Zeitbasis (B) angeschlossen ist, um ein Signal (BM) zu erhalten, das dem Verhältnis (I/E) der Einatmungszeit zur Ausatmungzeit entspricht, damit mit Hilfe der Leistungs- und Verhältnisangaben Signale für die Öffnungen der Magnetventile ($EV_A$, $EV_O$) so erarbeitet werden, dass während der Einatmungszeit jedes Magnetventil ($EV_A$, $EV_O$) eine Gasmenge durchlässt, die der mittleren Durchflussleistung während der Beatmungsperiode entspricht.

2. Vorrichtung nach Anspruch 1, dadurch gekennzeichnet, dass jeder Signalgeber ($G_A$, $G_O$) ein Schnellrelais vom Typ mit analogem Tor aufweist, dessen Öffnen von der Zeitbasis (B) gesteuert wird.

3. Vorrichtung nach Anspruch 1 oder 2, dadurch gekennzeichnet, dass die Organe des Mischers (M) zur Bestimmung der Durchflussleistung und des Sauerstoffanteils aus Potentiometern ($P_1$, $P_2$) bestehen, die so geschaltet sind, dass sie Spannungen liefern, die im Verhältnis zu den gewünschten Luft, und Sauerstoff-Durchflussleistungen stehen und die Schaltung analog ist und Werte ergibt, die für die gesamte Durchflussleistung und somit für die Luft- und Sauerstoff-Durchflussleistung während der Einatmungszeit kennzeichnend sind.

4. Vorrichtung nach Anspruch 1 oder 2, dadurch gekennzeichnet, dass die Organe des Mischers (M) zur Bestimmung der Durchflussleistung und des Sauerstoffanteils aus Potentiometern ($P_1$, $P_2$) bestehen, die so geschaltet sind, dass sie Spannungen liefern, die im Verhältnis zu den gewünschten Luft- und Sauerstoff-Durchfluss-leistungen stehen und die Schaltung logisch ist und Werte ergibt, die für die gesamte Durchflussleistung und somit für die Luft- und Sauerstoff-Durchflussleistung während der Einatmungszeit kennzeichnend sind.

Fig.1

Fig.2

*Fig.3*

*Fig.4*

Fig. 5

○ POINT TEST
✖ CONNECTOR TERMINAL
VOTAGE SUPPLY ±15V

0 032 349